# FASCICULE DE BREVET EUROPEEN

(11) **EP 0 875 264 B1**
(45) Date de publication et mention de la délivrance du brevet: **17.12.2003**
(21) Numéro de dépôt: 98401029.8
(22) Date de dépôt: 28.04.1998
(51) Int. Cl.: A61N 1/362, A61N 1/368

(54) **Stimulateur cardiaque/défibrillateur implantable avec double chambre protégé contre les phénomènes d'écoute croisée atrio-ventriculaire**
Implantierbarer Zweikammer- Herzschrittmacher/Defibrillator mit atrio-ventrikulärem Übersprechschutz
Double chamber implantable pacemaker/defibrillator with AV cross-talk protection

(30) Priorité: 29.04.1997 FR 9705286
(43) Date de publication de la demande: 04.11.1998
(73) Titulaire: ELA MEDICAL (Société anonyme), 92541 Montrouge (FR)
(72) Inventeur: Bonnet, Jean-Luc, 92120 Montrouge (FR); Geroux, Laurence, 92290 Chatenay Malabry (FR)
(74) Mandataire: Dupuis-Latour, Dominique

(56) Documents cités:
- EP-A- 0 562 237
- EP-A- 0 705 620
- EP-A- 0 830 877
- FR-A- 2 668 372
- US-A- 4 967 746
- US-A- 5 226 415
- US-A- 5 247 929

## Description

L'invention concerne les "dispositifs médicaux implantables actifs" tels que définis par la Directive 90/385/CEE du 20 juin 1990 du Conseil des communautés européennes, plus précisément les dispositifs stimulateurs cardiaques, défibrillateurs et/ou cardioverteurs pourvus d'une fonction de stimulation double chambre pour le traitement des troubles du rythme auriculaire.

Ces appareils, qui recueillent et délivrent des signaux aussi bien dans l'oreillette que le ventricule, sont sujets à un inconvénient connu sous le nom de "cross-talk" ou "écoute croisée".

Ce phénomène se produit lorsque, dans la cavité auriculaire, le stimulateur détecte un signal issu de la dépolarisation consécutive à une stimulation ventriculaire antérieure (cette écoute croisée sera désignée dans la suite "CTVA" ("Cross-Talk Ventriculo-Auriculaire")). Le dispositif peut interpréter à tort ce signal comme résultant d'une dépolarisation spontanée de l'oreillette, de sorte que ce phénomène est bien entendu préjudiciable au fonctionnement attendu du dispositif.

Dans les appareils actuels, ce phénomène est d'autant plus accentué qu'est grande la sensibilité des amplificateurs d'entrée de l'étage auriculaire ; cette sensibilité élevée est recherchée pour pouvoir recueillir et interpréter des signaux de faible amplitude de l'oreillette en cas de TdRA (Troubles du Rythme Auriculaire), terme générique qui recouvre diverses arythmies auriculaires telles que tachycardie, fibrillation, *flutter*, etc., troubles tous caractérisés, à la détection, par un rythme auriculaire anormal rapide.

Pour améliorer à la fois la sensibilité et la sélectivité de l'écoute et de la détection auriculaire, il est donc nécessaire de remédier aux conséquences de cette écoute croisée.

Une première solution consiste à créer un intervalle de temps appelé "blanking" durant lequel l'amplificateur auriculaire est complètement déconnecté du circuit d'écoute durant une stimulation ventriculaire (comme décrit par exemple dans le US-A-4 825 870 pour un amplificateur de signaux détectés dans la cavité ventriculaire).

Mais cette période de blanking n'est déclenchée que lors des stimulations et n'a qu'une courte durée ; celle-ci ne protège donc pas contre la détection tardive du signal de dépolarisation.

Le EP-A-0 594 957 décrit une autre manière de traiter le problème du CTVA. Il propose d'analyser les signaux recueillis et, quand un signal dans l'oreillette est détecté en même temps qu'est présent un signal sur l'étage ventriculaire, le dispositif déduit de cette concomitance que le signal auriculaire détecté a son origine dans le ventricule.

Une telle manière de procéder souffre cependant de l'inconvénient de ne pouvoir détecter des signaux spontanés émis par les deux cavités mais qui seraient totalement désynchronisés. De plus, un tel traitement des signaux ne considère que les seuls événements spontanés, à l'exception des événements stimulés.

Une autre solution serait d'augmenter la période réfractaire auriculaire. Cette solution, facile à mettre en oeuvre dans la plupart des stimulateurs actuels, aurait l'inconvénient de diminuer la fenêtre d'écoute auriculaire. Les arythmies auriculaires rapides pourraient alors être partiellement détectées, limitant l'efficacité des algorithmes de diagnostic.

Par ailleurs, le EP-A-0 705 620 décrit une technique de fenêtre dynamique afin de mieux reconnaître des signaux issus d'une tachycardie et les distinguer de ceux provenant d'un CTVA.

La présente invention a pour but de pallier les inconvénients décrits plus haut.

Essentiellement, l'invention propose de reconnaître le phénomène de CTVA en observant la manière dont se répètent les séquences stimulation ventriculaire/détection auriculaire. Une répétition de telles séquences entraînera une phase de suspicion de l'écoute croisée, qui déclenchera elle-même une phase de vérification ; si la vérification est positive, alors le dispositif prendra les mesures permettant de s'affranchir de ce phénomène.

Plus précisément, l'invention propose un dispositif médical implantable actif, notamment un stimulateur cardiaque, défibrillateur et/ou cardioverteur, pourvu d'une fonction de stimulation double chambre, ce dispositif comportant des moyens de détection d'événements cardiaques, spontanés ou stimulés, dans une première cavité et des moyens de stimulation dans une seconde cavité, et des moyens de détection et de confirmation d'écoute croisée, pour détecter dans la première cavité un signal issu d'une dépolarisation consécutive à une stimulation antérieure de la seconde cavité, et pour distinguer un tel signal d'un signal résultant d'une contraction spontanée de la première cavité.

Selon l'invention, les moyens de détection et de confirmation d'écoute croisée comprennent des moyens pour détecter une activité de type extra-systole de la première cavité survenant entre une stimulation de la seconde cavité et un événement consécutif, stimulé ou spontané, dans la première cavité.

La première cavité peut notamment être une cavité auriculaire et la seconde cavité, une cavité ventriculaire associée.

Les moyens de détection et de confirmation d'écoute croisée comprennent avantageusement des moyens pour détecter une extra-systole de la première cavité survenant entre une stimulation de la seconde cavité et un événement consécutif, stimulé ou spontané, dans la première cavité, pour déterminer que l'intervalle de temps compris entre la stimulation de la seconde cavité et l'extra-systole de la première cavité est inférieur à une durée prédéterminée, et pour détecter la stabilité de l'intervalle de temps compris entre la stimulation de la seconde cavité et l'extra-systole de la première cavité. Ces derniers moyens détecteurs de stabilité comprennent de préférence des moyens pour faire varier le délai entre la détection d'un événement dans la première cavité et la stimulation de la seconde cavité, et pour détecter l'absence de modification significative, à l'égard de ces variations, de l'intervalle de temps compris entre la stimulation de la seconde cavité et l'extra-systole de la première cavité.

Le dispositif peut en outre comprendre des moyens suppresseurs d'écoute croisée, qui accroissent la période réfractaire associée à la première cavité en cas de détection et de confirmation d'une écoute croisée ou, en variante, réduire la sensibilité des moyens de détection d'événements cardiaques dans la première cavité en cas de détection et de confirmation d'une écoute croisée.

Il prévoit également des moyens pour détecter la fin du phénomène d'écoute croisée et pour restaurer à leur valeur initiale les paramètres du dispositif modifiés par les moyens de détection et de confirmation d'écoute croisée.

On va maintenant décrire un exemple de réalisation détaillée de l'invention, en référence aux dessins annexés.

Les figures 1A et 1B illustrent, sur un tracé d'électrocardiogramme, la position des différents délais et périodes réfractaires mis en oeuvre.

Les figures 2 et 3 sont des organigrammes exposant l'enchaînement des diverses étapes du procédé mis en oeuvre par l'invention.

Dans l'exposé qui va suivre, on considérera le cas d'une sur-détection auriculaire. Toutefois, il serait possible d'envisager également le cas d'une sur-détection dans la cavité ventriculaire, le raisonnement présenté s'appliquant de la même façon, *mutatis mutandis*.

Comme on l'a indiqué plus haut, un CTVA est une sur-détection auriculaire : après une stimulation ventriculaire, dans certaines conditions, on peut détecter sur l'électrode auriculaire un signal qui n'est en fait qu'un "résidu" de la stimulation ventriculaire. Or cette détection se produit juste après la Période Réfractaire Absolue Auriculaire (PRAA), qu'il peut être souhaitable de programmer courte afin d'optimiser l'écoute auriculaire en cas de survenance d'arythmie auriculaire.

Ces détections auriculaires, en l'absence de traitement particulier, sont alors considérées, à tort, comme des événements prématurés pouvant faire suspecter un trouble du rythme auriculaire.

Le but de l'algorithme de l'invention est d'assurer une protection contre les CTVA :
- en diagnostiquant la survenance du CTVA et en opérant une différenciation entre CTVA et TdRA (Trouble du Rythme Auriculaire), et
- en maintenant dans la mesure du possible une détection optimale des arythmies auriculaires en évitant d'appliquer une période réfractaire auriculaire longue en l'absence de CTVA.

L'algorithme qui permet de réaliser ces fonctions comporte plusieurs phases :
1°) phase de détection, où l'on recherche la présence d'un CTVA éventuel,
2°) phase de confirmation, pour discriminer CTVA et TdRA,
3°) phase de suppression, pour protéger l'appareil en cas de CTVA avéré.
4°) phase de restauration des paramètres initiaux à la disparition du CTVA, afin de ne pas pénaliser l'écoute auriculaire en cas de disparition du CTVA.

On va maintenant décrire en détail ces phases :

### 1°) phase de détection du CTVA

En fonctionnement normal, l'appareil enregistre la séquence des événements ventriculaires, en particulier le signal de stimulation ventriculaire, et la séquence des événements auriculaires (par "événement" on entendra soit les signaux provenant de dépolarisations spontanées, soit des signaux résultant de stimuli appliqués par l'appareil).

Le dispositif comprend des moyens de détection des ESA (Extra-Systoles Auriculaires) et des ESV (Extra-Systoles Ventriculaires) comme décrit par exemple dans le EP-A-0 676 217 (ELA Médical) : une onde P (recueil d'une activité ayant son origine dans l'oreillette) est définie comme une ESA si l'intervalle de temps séparant cette onde P du précédent événement auriculaire est inférieur à une fraction de l'intervalle PP moyen, c'est-à-dire l'intervalle moyen de la fréquence auriculaire calculé sur (typiquement) huit cycles cardiaques ne comprenant pas d'extra-systole.

L'algorithme de l'invention a pour objet de repérer, dans la séquence des signaux, les ondes P précoces ressemblant à des ESA. Dans les phases ultérieures, on déterminera s'il s'agit de véritables ESA, c'est-à-dire de signaux révélateurs d'un TdRA par accélération anormale du rythme, ou d'un CTVA, donc d'un signal parasite. Dans ce dernier cas, et seulement dans ce cas, on prendra les mesures nécessaires pour s'affranchir du caractère parasite de ce CTVA.

Le CTVA se caractérise par une succession de séquences V-ESA-A ou V-ESA-P, ces motifs se répétant tant que dure le CTVA, et éventuellement avec une alternance de ces deux motifs ("V" = onde ventriculaire stimulée ; "A" = onde auriculaire stimulée ; "P" = onde auriculaire sinusale spontanée).

On peut définir trois caractéristiques révélatrices d'un CTVA :
a) présence d'une ESA par cycle ventriculaire, pour chacun des deux motifs ;
b) stabilité de l'intervalle V-ESA, car cet intervalle ne dépend que de la dépolarisation ventriculaire (le critère de stabilité peut être le même que celui que l'on considère lorsque l'on souhaite détecter des Tachycardies induites par Ré-Entrée (TRE), comme cela est décrit par exemple dans les FR-A-2 54 988 et FR-A-2 668 372, que l'on évoquera par la suite plus en détail) ;
c) intervalle V-ESA court, de l'ordre de 100 à 200 ms.

Dans cette phase de détection, on va observer un certain nombre de cycles ventriculaires consécutifs, en cherchant si ces trois critères sont vérifiés.

Toutefois, si ces caractéristiques sont nécessaires pour que l'on soit en présence de CTVA, elles ne sont pas suffisantes. En effet, certains troubles du rythme auriculaire, tels que tachycardie ou "flutter" auriculaire, présentent également ces caractéristiques, le stimulateur fonctionnant dans ce cas en mode 2:1 ou 3:1.

La phase suivante dite "de confirmation" va permettre d'effectuer cette discrimination.

### 2°) Phase de confirmation du CTVA

Pour ce faire, on peut procéder en modulant le délai atrio-ventriculaire (DAV), car le CTVA est lié à la stimulation ventriculaire,.

Cette technique est en elle-même connue dans une autre application, qui est celle des algorithmes anti-TRE, comme décrits dans les FR-A-2 54 988 et FR-A-2 668 372 précités, où après une phase de suspicion de TRE, on module le DAV afin de confirmer que le trouble observé est bien une TRE et non un TdRA.

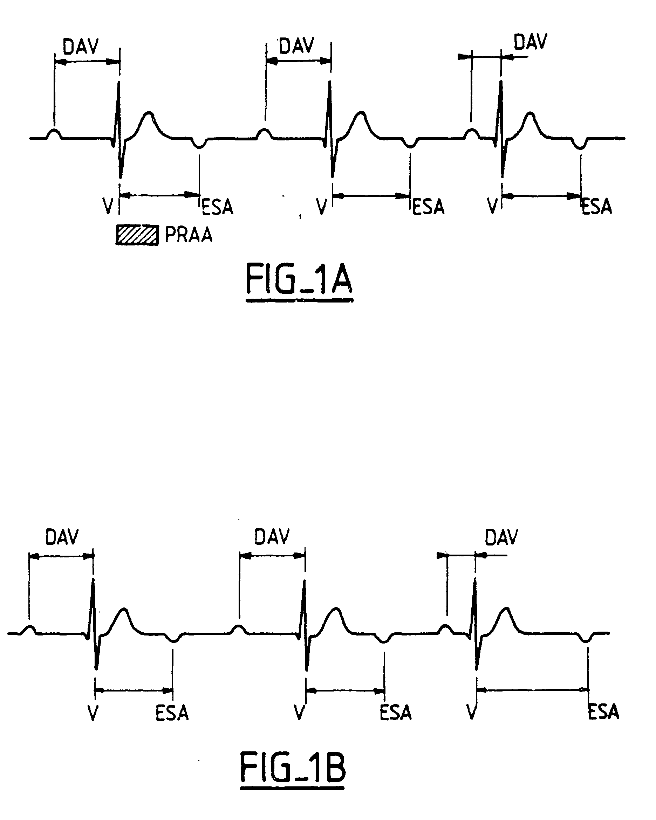

En appliquant cette technique au cas présent, dès que la phase de détection a conduit à une suspicion de CTVA, le stimulateur module le DAV afin de déterminer si l'intervalle de temps V-ESA reste stable ou varie : dans le premier cas (cf. figure 1A), il s'agit d'un CTVA ; dans le cas contraire (cf. figure 1B) il s'agit d'un TdRA.

La modulation du DAV dépend de l'état dans lequel se trouve le stimulateur. Si le DAV programmé par l'appareil à l'instant considéré le permet, la modulation du DAV est négative, c'est-à-dire que le DAV doit être diminué. Dans le cas contraire, le DAV est augmenté.

Si l'intervalle de temps V-ESA reste stable en dépit de cette modulation du DAV, on confirme la présence du CTVA, et l'on passe à la phase suivante qui a pour objet de protéger l'appareil contre cet événement parasite.

### 3°) Phase de suppression du CTVA

Dès confirmation d'un CTVA, le stimulateur modifie son comportement de façon à ce que le CTVA ne puisse pas être interprété comme résultant d'une contraction spontanée de l'oreillette.

Une première solution consiste à modifier les périodes réfractaires définies par l'appareil en ajoutant une période réfractaire relative Pctva telle que tout événement auriculaire détecté dans cette fenêtre soit considéré comme n'étant pas une ESA et ne soit pas enregistré comme une ESA.

Cette période réfractaire Pctva est par exemple égale à l'intervalle [V-ESA] + 31 ms, et doit être relative pour permettre la détection et l'analyse des CTVA.

En variante, une autre solution pour protéger l'appareil contre les conséquences du CTVA consiste à simplement baisser la sensibilité de la détection auriculaire, en augmentant le seuil de détection jusqu'à disparition du CTVA.

### 4°) Phase de restauration après CTVA

Le retour au fonctionnement normal peut intervenir :
- soit par disparition spontanée du CTVA,
- soit par test à intervalle régulier de l'existence du CTVA pour savoir si celui-ci est toujours présent,
- soit par retour à une activité V spontanée.

### Organigramme détaillé de mise en oeuvre

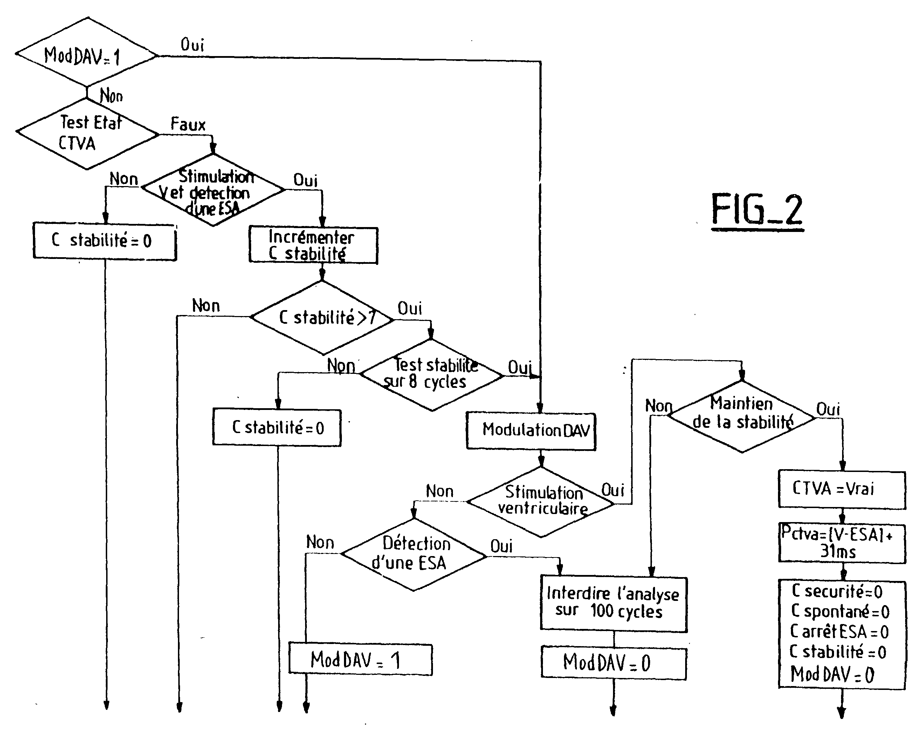

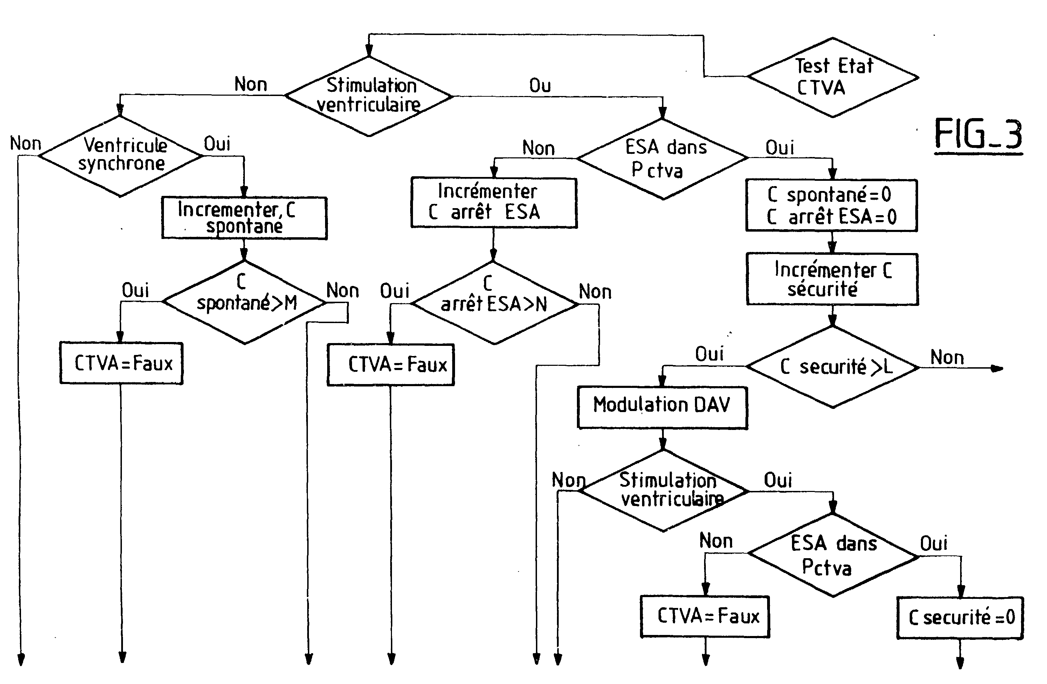

Les figures 2 et 3, dont on considérera qu'elles font partie de la présente description, donnent un organigramme détaillé de mise en oeuvre de l'algorithme dont on vient d'exposer ci-dessus les grandes lignes, la figure 2 correspondant aux phases de détection et de confirmation des CTVA et la figure 3 à la phase de suppression.

Les différentes variables et abréviations utilisées sur ces organigrammes sont les suivantes :
- "CTVA" : Variable binaire, indiquant la présence ou l'absence de Cross-talk VA.
- "Pctva" : Période réfractaire de recouvrement du CTVA, typiquement Pctva = [V-ESA] + 31 ms.
- "Cstabilité" : Compteur permettant de tester la stabilité de la détection du CTVA sur 8 cycles ; ce compteur sert uniquement à compter huit cycles consécutifs comportant une stimulation ventriculaire suivie d'une détection auriculaire réfractaire (ESA).
- "Csécurité" : Compteur de sécurité, permettant après le diagnostic d'un CTVA de tester périodiquement la persistance du phénomène ; donc, tous les 'Csécurité' cycles ventriculaires, on module à nouveau le DAV afin de voir si le phénomène est toujours présent.
- "Cspontané" : Compteur de sortie par réapparition du rythme spontané : au bout de 'Cspontané' cycles ventriculaires spontanés, on sort de l'algorithme ; en effet il n'y a plus de CTVA si le ventricule n'est plus stimulé.
- "CarrêtESA" : Compteur de sortie par disparition du CTVA : au bout de 'CarrêtESA' cycles ventriculaires stimulés sans CTVA détecté en Pctva, on sort de l'algorithme car le phénomène a disparu de façon prolongée.
- "Stimulation V et détection d'une ESA" : Cycle avec une stimulation ventriculaire et détection d'un événement auriculaire en période réfractaire relative (donc d'une ESA).
- "Test stabilité sur 8 cycles" : Sur les huit cycles on a eu à chaque cycle une stimulation ventriculaire suivie d'un détection auriculaire en période réfractaire (donc d'une ESA). Avec ce test, on veut voir si le délai entre la stimulation ventriculaire et la détection auriculaire est stable sur les huit cycles.
- "Modulation du DAV" : Pour confirmer le CTVA. on veut vérifier que la stabilité du délai entre la stimulation ventriculaire et détection auriculaire dépend uniquement de la stimulation ventriculaire et non de l'événement auriculaire précédent. Pour cela, on module le DAV afin de changer le délai entre l'événement auriculaire précédent et la détection ventriculaire.
- "Maintien de la stabilité" : Après la modulation, on regarde si le délai entre la stimulation ventriculaire et la détection auriculaire est toujours identique.

### Recherche du CTVA - figure 2

Le diagnostic de CTVA se fait en deux étapes, la première étant la recherche d'un intervalle [V-ESA] stable. Pour ceci, on doit avoir sur huit cycles ventriculaires :
- des stimulations ventriculaires à chaque cycle,
- une ESA et une seule à chaque cycle,
- un intervalle [V-ESA] stable, typiquement à 16 ms près sur les huit cycles.

La seconde étape est la modulation du DAV : dès que les conditions ci-dessus sont remplies, on effectue sur le cycle suivant une modulation du DAV pour vérifier que la stabilité de l'intervalle VP ne dépend pas du signal auriculaire précédent mais uniquement de la stimulation ventriculaire.

La modulation sera négative et de 31 ms (par exemple) si le DAV le permet, sinon la modulation sera positive et de 31 ms (par exemple).

Dans un premier temps, on valide la modulation du DAV :
- si une détection ventriculaire survient suite à la modulation :
   . s'il y a une ESA après cet événement ventriculaire, alors le diagnostic de CTVA est négatif (c'est probablement un TdRA).
   . s'il n'y a pas d'ESA alors l'étape de modulation du DAV est réitérée au prochain cycle car aucune décision ne peut être prise.
- dans le cas d'une détection ventriculaire asynchrone au cours du cycle où l'on aurait dû moduler, alors la modulation est reportée au cycle suivant et l'analyse se poursuit normalement.

Si aucun des deux cas précédents n'est avéré, l'intervalle [V-ESA] est analysé sur le cycle modulé :
- si, suite à la modulation, le ventricule est stimulé et l'intervalle [V-ESA] est identique à l'intervalle calculé sur les 8 cycles (à 16 ms près), alors le diagnostic de CTVA est positif.
- si, suite à la modulation, le ventricule est stimulé et l'intervalle [V-ESA] est différent de celui mesuré sur les 8 cycles précédents (à 16 ms près) alors le diagnostic de CTVA est négatif et la recherche de CTVA est interdite pendant 100 cycles.

Ensuite, si le diagnostic de CTVA est effectué (diagnostic positif) :
- on calcule la valeur d'une fenêtre de recouvrement du CTVA : Pctva = [V-ESA] + 31 ms, on initialise un compteur Csécurité permettant de contrôler régulièrement si le diagnostic de CTVA est toujours vérifié.
- on initialise un compteur CarrêtESA qui conditionnera la sortie du diagnostic de CTVA en cas de disparition des détections en Pctva.
- on initialise un compteur Cspontané permettant de conditionner la sortie du diagnostic de CTVA en cas d'apparition d'une conduction spontanée.

### Phase de restauration (fonctionnement en état CTVA et diagnostic de fin de CTVA) - figure 3

Lorsque l'appareil a détecté un CTVA le fonctionnement général est le même, notamment, tous les algorithmes sont actifs. Seules les périodes réfractaires changent. En effet, on ajoute aux périodes précédentes la période réfractaire relative de CTVA désignée Pctva (voir figure 1).

Cette période réfractaire relative n'est appliquée qu'après une stimulation ventriculaire. Elle n'est plus appliquée après une détection ventriculaire, synchrone ou non.

La survenance du CTVA est testée tous les cycles ventriculaires une fois que le diagnostic de CTVA a été effectué, ceci afin de favoriser un retour rapide en fonctionnement normal avec une période réfractaire optimisée. Cette recherche n'est effectuée qu'en période d'association AV en 1:1.

*Test périodique de persistance du CTVA* : L'objet de ce test est de vérifier, une fois que le CTVA est installé, qu'aucune erreur de diagnostic ne s'est produite au début de la recherche. Pour cela, on effectue périodiquement un test de modulation. Si le ventricule est stimulé et qu'une onde P est détectée en Pctva alors on incrémente Csécurité. Dès que ce compteur dépasse une valeur L, par exemple L = 1000, on teste à nouveau la survenance d'un CTVA à l'aide d'une modulation. Le DAV est modulé de 31 ms, en négatif si possible, si après la modulation il y a toujours une détection P en Pctva alors CTVA reste à vrai et Csécurité est remis à zéro. S'il n'y a pas de détection P en Pctva alors CTVA est mis à faux et l'appareil retourne en phase de recherche de CTVA.

*Fin de CTVA par arrêt de la détection croisée* : Si le ventricule est stimulé et qu'aucune onde P n'est détectée en Pctva, alors on teste la possibilité de sortie de CTVA par arrêt du phénomène. Le compteur CarrêtESA est incrémenté et, dès que ce compteur dépasse une valeur N, par exemple N = 16, CTVA est mis à faux et l'appareil retourne en phase de recherche CTVA.

*Fin de CTVA par retour de la conduction spontanée* : Si le ventricule n'est pas stimulé et est synchronisé, alors on teste la possibilité de sortie de CTVA par retour de la conduction spontanée. Le compteur Cspontané est incrémenté et, dès que ce compteur dépasse une valeur M, par exemple M = 16, CTVA est mis à faux et l'appareil retourne en phase de recherche de CTVA (lorsque la conduction est spontanée, Pctva n'est pas appliquée).

Dès que la condition CTVA devient fausse, Pctva est remise à zéro et/ou la sensibilité auriculaire est remise à sa valeur initiale (valeur programmée).

Les deux compteurs de sortie Cspontané et CarrêtESA sont remis à zéro dès que réapparaît le phénomène de CTVA, c'est-à-dire une stimulation suivie d'une détection auriculaire en Pctva.

## Revendications

1. Un dispositif médical implantable actif, notamment un stimulateur cardiaque, défibrillateur et/ou cardioverteur, pourvu d'une fonction de stimulation double chambre, ce dispositif comportant des moyens de détection d'événements cardiaques, spontanés ou stimulés, dans une première cavité et des moyens de stimulation dans une seconde cavité, ainsi que des moyens de détection et de confirmation d'écoute croisée, pour détecter dans la première cavité un signal issu d'une dépolarisation consécutive à une stimulation antérieure de la seconde cavité, et pour distinguer un tel signal d'un signal résultant d'une activité spontanée de la première cavité,
dispositif **caractérisé en ce que** les moyens de détection et de confirmation d'écoute croisée comprennent des moyens pour détecter une activité de type extra-systole de la première cavité survenant entre une stimulation de la seconde cavité et un événement consécutif, stimulé ou spontané, dans la première cavité.

2. Le dispositif de la revendication 1, dans lequel la première cavité est une cavité auriculaire et la seconde cavité est une cavité ventriculaire associée.

3. Le dispositif de la revendication 1, dans lequel les moyens de détection et de confirmation d'écoute croisée comprennent des moyens pour déterminer que l'intervalle de temps compris entre la stimulation de la seconde cavité et l'extra-systole de la première cavité est inférieur à une durée prédéterminée.

4. Le dispositif de la revendication 1, dans lequel les moyens de détection et de confirmation d'écoute croisée comprennent des moyens pour détecter la stabilité de l'intervalle de temps compris entre la stimulation de la seconde cavité et l'extra-systole de la première cavité.

5. Le dispositif de la revendication 4, dans lequel les moyens détecteurs de stabilité comprennent des moyens pour faire varier le délai (DAV) entre la détection d'un événement dans la première cavité et la stimulation de la seconde cavité, et pour détecter l'absence de modification significative, à l'égard de ces variations, de l'intervalle de temps ([V-ESA]) compris entre la stimulation de la seconde cavité et l'extra-systole de la première cavité.

6. Le dispositif de la revendication 1, comprenant en outre des moyens suppresseurs d'écoute croisée, pour accroître (Pctva) la période réfractaire (PRAA) associée à la première cavité en cas de détection et de confirmation d'une écoute croisée.

7. Le dispositif de la revendication 1, comprenant en outre des moyens suppresseurs d'écoute croisée, qui accroissent la sensibilité des moyens de détection d'événements cardiaques dans la première cavité en cas de détection et de confirmation d'une écoute croisée.

8. Le dispositif de la revendication 1, comprenant en outre des moyens pour détecter la fin du phénomène d'écoute croisée et pour restaurer à leur valeur initiale les paramètres du dispositif modifiés par les moyens de détection et de confirmation d'écoute croisée.

## Patentansprüche

1. Implantierbare aktive medizinische Vorrichtung, insbesondere ein Herzschrittmacher, Defibrillator und/oder Cardioverter, ausgestattet mit einer Funktion zur Doppelkammer-Stimulation, wobei diese Vorrichtung Mittel zum Detektieren von Herz-Ereignissen, spontanen oder stimulierten, in einem ersten Hohlraum und Mittel zur Stimulation in einem zweiten Hohlraum aufweist, sowie Mittel zum Detektieren und Bestätigen von Übersprechen, um in dem ersten Hohlraum ein Signal zu erfassen, das von einer Depolarisation ausgeht, die einer vorhergehenden Stimulation des zweiten Hohlraums folgt, und um ein derartiges Signal von einem Signal zu unterscheiden, das von einer spontanen Aktivität des ersten Hohlraums herrührt, wobei die Vorrichtung **dadurch gekennzeichnet ist, dass** die Mittel zum Detektieren und zum Bestätigen des Übersprechens Mittel zum Detektieren einer Aktivität vom Typ Extrasystole von dem ersten Hohlraum umfassen, welche eintritt zwischen einer Stimulation des zweiten Hohlraums und einem folgenden Ereignis, stimuliert oder spontan, in dem ersten Hohlraum.

2. Vorrichtung gemäß Anspruch 1, in welcher der erste Hohlraum ein aurikularer Hohlraum und der zweite Hohlraum ein zugehöriger ventrikularer Hohlraum ist.

3. Vorrichtung gemäß Anspruch 1, in welchem die Mittel zum Detektieren und Bestätigen des Übersprechens Mittel zum Bestimmen umfassen, ob das zwischen der Stimulation des zweiten Hohlraums und der Extrasystole des ersten Hohlraums liegende Zeitintervall kleiner ist als eine vorgegebene Dauer.

4. Vorrichtung gemäß Anspruch 1, in welcher die Mittel zum Detektieren und Bestätigen des Übersprechens Mittel zum Detektieren der Stabilität des Zeitintervalls zwischen der Stimulation des zweiten Hohlraums und der Extrasystole des ersten Hohlraums umfassen.

5. Vorrichtung gemäß Anspruch 4, in welcher die Mittel zum Detektieren der Stabilität Mittel zum Veranlassen einer Variation der Verzögerung (DAV) zwischen der Detektion eines Ereignisses in dem ersten Hohlraum und der Stimulation des zweiten Hohlraums, und um die Abwesenheit einer bedeutenden Änderung zu detektieren, im Hinblick auf diese Variationen, des Zeitintervalls ([V-ESA]) zwischen der Stimulation des zweiten Hohlraums und der Extrasystole des ersten Hohlraums.

6. Vorrichtung gemäß Anspruch 1, ferner Mittel zum Unterdrücken von Übersprechen umfassend, um die Refraktärzeitspanne (PRAA) zu vergrößern (Pctva), die mit dem ersten Hohlraum assoziiert ist, im Fall der Erfassung und der Bestätigung von Übersprechen.

7. Vorrichtung gemäß Anspruch 1, ferner Mittel zum Unterdrücken von Übersprechen umfassend, welche die Sensibilität der Mittel zum Detektieren von Herz-Ereignissen in dem ersten Hohlraum vergrößern, im Fall der Detektion und der Bestätigung von Übersprechen.

8. Vorrichtung gemäß Anspruch 1, ferner Mittel zum Detektieren des Endes des Phänomens des Übersprechens umfassend, zum Wiederherstellen der Parameter der Vorrichtung auf ihren anfänglichen Wert, die durch die Mittel zum Erfassen und zum Bestätigen von Übersprechen verändert wurden.

## Claims

1. An active implantable medical device, especially a cardiac pacemaker, defibrillator and/or cardioverter, having a double-chamber stimulation function, this device comprising means for detecting cardiac spontaneous or stimulated events in a first cavity and means for stimulating in a second cavity, along with means for detection and confirmation of cross-sensing, to detect in the first cavity a signal coming from a depolarization consecutive to a preceding stimulation of the second cavity, and to distinguish such a signal from a signal resulting from spontaneous contraction of the first cavity,
said device being **characterized in that** the cross-sensing detection and confirmation means comprise means to detect an activity of the extra-systole type of the first cavity occurring between a stimulation of the second cavity and a consecutive event, stimulated or spontaneous, in the first cavity.

2. The device of claim 1, wherein the first cavity is an atrial cavity and the second cavity is an associated ventricular cavity.

3. The device of claim 1, wherein the cross-sensing detection and confirmation means comprise means for detecting that the interval of time between the stimulation of the second cavity and the extra-systole of the first cavity is less than a predetermined duration.

4. The device of claim 1, wherein the cross-sensing detection and confirmation means comprise means for detecting the stability of the time interval between the stimulation of the second cavity and the detected extra-systole of the first cavity.

5. The device of claim 4, wherein the stability detecting means comprise means for varying the period (DAV) between the detection of an event in the first cavity and the stimulation of the second cavity, and to detect the absence of significant modification, with regard to these variations, of the time interval ([V-ESA]) between the stimulation of the second cavity and the extra-systole of the first cavity.

6. The device of claim 1, further comprising means for suppressing cross-sensing, in order to increase (Pctva) the refractory period (PRAA) associated with the first cavity in case of detection and confirmation of a cross-sensing.

7. The device of claim 1, further comprising means for suppressing cross-sensing, which increase the sensitivity of the means for detecting cardiac events in the first cavity in case of detection and confirmation of a cross-sensing.

8. The device of claim 1, further comprising means for detecting the end of the cross-sensing phenomenon, and to restore to their initial values the parameters of the device modified by the cross-sensing detection and confirmation means.
